# EUROPEAN PATENT SPECIFICATION

(11) **EP 3 437 692 B1**
(45) Date of publication and mention of the grant of the patent: **26.08.2020**
(21) Application number: 17184492.1
(22) Date of filing: 02.08.2017
(51) Int. Cl.: A61N 1/36

(54) **METHOD FOR PREDICTION OF CLINICAL RESPONSE TO VNS THERAPY IN EPILEPTIC PATIENTS**
VERFAHREN ZUR VORHERSAGE DER KLINISCHEN REAKTION AUF VNS-THERAPIE BEI EPILEPSIEPATIENTEN
PROCÉDÉ DE PRÉDICTION DE LA RÉPONSE CLINIQUE À LA THÉRAPIE PAR SNV CHEZ DES PATIENTS ÉPILEPTIQUES

(43) Date of publication of application: 06.02.2019
(73) Proprietor: Masarykova Univerzita, 60177 Brno (CZ)
(72) Inventor: Brazdil, Milan, 62800 Brno (CZ); Dolezalova, Irena, 69002 Breclav (CZ); Koritakova, Eva, 59252 Rozna (CZ); Chrastina, Jan, 66448 Nebovidy u Brna (CZ); Roman, Robert, 60200 Brno (CZ); Chladek, Jan, 63500 Brno (CZ); Pail, Martin, 61400 Brno (CZ)
(74) Representative: Hartvichova, Katerina

(56) References cited:
- US-A- 5 611 350
- US-A1- 2005 043 774
- US-A1- 2007 150 025
- US-A1- 2012 330 369
- US-A1- 2015 142 082
- US-A1- 2016 310 070
- US-B1- 6 549 804
- DE VOS C C ET AL: "Predicting success of vagus nerve stimulation (VNS) from interictal EEG", SEIZURE, BAILLIERE TINDALL, LONDON, GB, vol. 20, no. 7, 1 April 2011 (2011-04-01), pages 541-545, XP028247496, ISSN: 1059-1311, DOI: 10.1016/J.SEIZURE.2011.04.002 [retrieved on 2011-04-07]
- MAJOIE H J M ET AL: "Vagus nerve stimulation in patients with catastrophic childhood epilepsy, a 2-year follow-up study", SEI, BAILLIERE TINDALL, LONDON, GB, vol. 14, no. 1, 1 January 2005 (2005-01-01), pages 10-18, XP004707538, ISSN: 1059-1311, DOI: 10.1016/J.SEIZURE.2004.02.003

## Description

### Field of Art

The present invention relates to a method for prediction of clinical response to vagal nerve stimulation in a patient suffering from epilepsy, using data from routine EEG.

### Background Art

Resective surgery is currently the best therapeutic option for treatment of patients with drug-resistant epilepsy. However, there is still a substantial amount of intractable patients, who are not eligible for resective surgery or in whom resective surgery failed to abolish seizures. Chronic vagal nerve stimulation (VNS) is a well-established palliative method, introduced into the treatment of drug-resistant epilepsy in 1994. It rarely results in complete seizure-freedom (∼ 5% of treated subjects), but can offer the possibility for substantial (i.e. ≥ 50%) seizure reduction in approx. 50-60 % of patients. On the other hand, the seizure frequency remains unchanged in about 25 % of patients on VNS therapy.

A reliable method of identification of patients who would profit from VNS therapy would bring substantial benefits in improved patient selection, minimization of useless surgery procedures and reduction of financial expenses. Unfortunately, currently no such method is available for prediction of individual efficacy of this treatment prior to implantation of the VNS device. Recent meta-analysis of VNS efficacy in epilepsy just revealed a statistically significant trend toward a greater benefit in pediatric patients, in patients with generalized epilepsies, posttraumatic epilepsies and individuals with tuberous sclerosis (Englot DJ, Chang EF, Auguste KI. Vagus nerve stimulation for epilepsy: a meta-analysis of efficacy and predictors of response. J Neurosurg 2011;115: 1248-55). However, there is no procedure available which would provide a pre-operative information which individual patient would be a VNS responder (i.e. patients with ≥ 50% seizure reduction) and which one would be a non-responder (< 50% seizure reduction).

Recently, several authors aimed to identify predictors of VNS outcome. Despite a significant effort, clinical predictors of individual responsiveness to VNS therapy are still elusive. Few studies used the electroencephalogram (EEG) as a tool for assessment of outcome prognosis (Janszky J, Hoppe M, Behne F, Tuxhorn I, Pannek HW, Ebner A. Vagus nerve stimulation: predictors of seizure freedom. J Neurol Neurosurg Psychiatry 2005;76: 384-9; Majoie HJ, Berfelo MW, Aldenkamp AP, Renier WO, Kessels AG. Vagus nerve stimulation in patients with catastrophic childhood epilepsy, a 2-year follow-up study. Seizure 2005;14: 10-8; de Vos CC, Melching L, van Schoonhoven J, Ardesch JJ, de Weerd AW, van Lambalgen HC, van Putten MJ. Predicting success of vagus nerve stimulation (VNS) from interictal EEG. Seizure 2011;20: 541-5; De Taeye L, Vonck K, van Bochove M, Boon P, Van Roost D, Mollet L, Meurs A, De Herdt V, Carrette E, Dauwe I, Gadeyne S, van Mierlo P, Verguts T, Raedt R. The P3 event-related potential is a biomarker for the efficacy of vagus nerve stimulation in patients with epilepsy. Neurotherapeutics 2014; 11: 612-22; Wostyn S, Staljanssens W, De Taeye L, Strobbe G, Gadeyne S, Van Roost D, Raedt R, Vonck K, van Mierlo P. EEG Derived Brain Activity Reflects Treatment Response from Vagus Nerve Stimulation in Patients with Epilepsy. Int J Neural Syst 2016: 1650048.2-6). In these investigations however, limited sample size and/or analyzing EEG in already implanted patients during VNS ON and OFF conditions, greatly question practical impact of authors' results.

### Disclosure of the Invention

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

The present invention provides a method for determining of clinical response of a patient suffering from epilepsy to chronic vagal nerve stimulation (VNS) therapy, comprising the steps of:
- obtaining theta, alpha, beta and gamma frequency bands for at least one, preferably at least 10 scalp electrodes from scalp EEG data obtained from the patient using an EEG recording protocol, said EEG data comprising at least one rest interval, preferably at the beginning of the EEG data, at least one open eyes/close eyes interval, at least one photic stimulation interval, at least one hyperventilation interval, and optionally at least one additional open eyes/close eyes interval and optionally at least one rest interval having the duration of at least 30 s at the end of the EEG data, wherein one interval is selected as a baseline interval,
- obtaining absolute mean powers as mean values of passband power envelope inside at least one discriminative interval and inside the baseline interval, and obtaining relative mean power of the at least one discriminative interval as the ratio of absolute mean power of said discriminative interval relative to absolute mean power of the baseline interval,
- determining from the relative mean powers of the at least one discriminative interval for at least one discriminative electrode whether the patient is a responder or a non-responder to vagal nerve stimulation therapy based on a responder pattern or a non-responder pattern, wherein the responder and non-responder patterns are determined by statistical analysis of EEG data measured using the said EEG recording protocol for a group of known responders and non-responders.

The discriminative electrodes can be determined by the user (e.g., a medical practicioner or an EEG-reading specialist, e.g., based on previous experience), or they can be obtained by statistical analysis of EEG data measured using the said EEG recording protocol for a group of known responders and non-responders.

The discriminative intervals can be determined by the user (e.g., a medical practicioner or an EEG-reading specialist, e.g., based on previous experience), or they can be obtained by statistical analysis of EEG data measured using the said EEG recording protocol for a group of known responders and non-responders.

Standard scalp EEG is commonly recorded for each epilepsy patient already when diagnosing epilepsy. Thus, there is no need for a separate recording of routine EEG for performing this method. Scalp EEG recording is an established standard procedure, typically lasting 20-40 minutes and involving recording from electrodes placed on the scalp of the patient. Standard scalp EEG records the cerebral electrical activity on the surface of head. Each region of the brain is characterized by its ictal activity in different frequency bands.

EEG data are data describing the dependency of cerebral electrical activity on time. When the EEG is recorded, using scalp electrodes detecting the brain electrical activity, the patients are asked to rest or to do various activities (typically opening and closing eyes, hyperventilating) or are stimulated (e.g. photic stimulation). Intervals of the EEG data are typically named in accordance with these rests, activities or stimulations. The word "interval" when used in connection with the EEG data designates a portion of the data representing the EEG signal recorded during a temporal interval of the measurement in which said rests, activities or stimulations were performed.

The scalp EEG data used in the present invention are data obtained from the patient before the VNS therapy or VNS implantation. Typically, data from routine scalp EEG recording, which is done for each patient during or after epilepsy diagnosis, may be used in the method of the present invention.

The EEG data typically comprise at least one rest interval, preferably at the beginning of the EEG data, at least one open eyes/close eyes interval, at least one photic stimulation interval, at least one hyperventilation interval, and optionally at least one additional open eyes/close eyes interval and optionally at least one rest interval having the duration of at least 30 s at the end of the EEG data. These intervals may generally follow in any order in the EEG data, but typically one or more of the following is fulfilled: one rest interval is at the beginning of the EEG data, at least one rest interval is at the end of the EEG data, an open eyes/close eyes interval follows the rest interval at the beginning of the EEG data, open eyes/close eyes interval is followed by a rest interval, each interval has a temporal length of 8 seconds to 5 minutes, intervals apart from hyperventilation have a temporal length of 8 seconds to 3 minutes.

One of the intervals is selected as a baseline interval, typically the baseline interval is a rest interval, more preferably a rest interval at the beginning of the EEG data or at the end of the EEG data.

The photic stimulation is usually performed using flashing light device emitting photic light (flash light) where intensity, frequency and duration of the emitted light are operator controlled. The flash light is positioned about a meter distance from the patient head and usually flash light is not given continuously when frequency is changed as a pause for a certain time separates different flash light frequency values. The photic stimulation is usually used as a part of routine EEG test.

In hyperventilation, patient is instructed to breath with the maximum of effort. Typically, the hyperventilation lasts for about 3 minutes and is done once or twice during EEG recording.

The theta, alpha, beta and gamma frequency bands are generally known in the field of art of encephalography. The theta band typically corresponds to 4-7.5 Hz ± 1 Hz, the alpha band corresponds to 8-12 Hz ± 1 Hz, the beta band corresponds 14-30 Hz ± 1 Hz, and the gamma band corresponds to 31-45 Hz ± 1 Hz.

The theta, alpha, beta and gamma frequency bands are preferably obtained for at least 13 or at least 16 scalp electrodes. Preferably, the theta, alpha, beta and gamma frequency bands are obtained for all scalp electrodes according to 10-20 international system.

The "EEG recording protocol" should be understood as a set of conditions characterizing the EEG recording, such as the type of the EEG apparatus, the sequence of the rests, activities and stimulations (which translates into the sequence of intervals in the EEG data), the filters used for filtering the data during or after the recording, etc. Each EEG-recording center consistently uses the same recording protocol, which however differs to a certain extent from recording protocols used by other EEG-recording centers.

As the EEG recording protocol differs to a certain extent between the individual EEG-recording centers, the data recorded by a specific EEG-recording center should be evaluated using discriminative electrodes, discriminative intervals and responder and non-responder patterns obtained by statistical analysis of known data obtained from that EEG-recording center.

The "discriminative electrode" should be understood as an electrode or a group of electrodes the EEG data from which differs for responders and non-responders in at least one interval with statistical significance.

The "discriminative interval" should be understood as an interval in which the EEG data differs for responders and non-responders for at least one electrode with statistical significance.

The "responder pattern" should be understood as a set of values of relative mean powers in the discriminative intervals for the discriminative electrodes which have been determined by statistical analysis as determining a patient suffering from epilepsy who responds to VNS therapy, preferably with accuracy as determined by the statistical model.

The "non-responder pattern" should be understood as a set of values of relative mean powers in the discriminative intervals for the discriminative electrodes which have been determined by statistical analysis as determining a patient suffering from epilepsy who does not respond to VNS therapy, preferably with accuracy as determined by the statistical model.

In one embodiment, a responder is defined as a patient in whom the VNS therapy results in ≥ 50% seizure reduction. In this embodiment, a non-responder is defined as a patient in whom the VNS therapy results in less than 50% seizure reduction. However, different seizure reduction percentage levels may be used for definition of responders or non-responders.

In one embodiment, the term "discriminative electrode" may mean a single electrode, i.e., the assessment of mean powers is done at the level of single electrodes. Alternatively, the term "discriminative electrode" may mean a group of electrodes, i.e., the assessment of mean powers is done at the level of groups of electrodes, e.g., using mean value from all electrodes of the group. A group of electrodes may comprise, for instance, electrodes placed in one anatomical area. The anatomical areas are: frontal (left, right), temporal (left, righ), cetral, parietal (left, right), occipital (left, right). In the international 10-20 system, the groups of electrodes grouped by anatomical areas are: (1) Fp1, F3, Fz; (2) Fp2, F4, Fz; (3) F7, T3; (4) F8, T4; (5) C3, Cz, C4; (6) P3, Pz, T5, O1; (7) P4, Pz, T6, O2. Electrodes may also be grouped differently than by anatomical areas.

Within the framework of this invention, it was found that VNS responders and non-responders significantly differ in EEG power within the theta, alpha, beta and gamma frequency bands. This is the first VNS-response prediction method showing that evaluation of routine-recorded EEG data are useful for identification of patients with favourable VNS therapy outcome (responders) and patients who would not benefit from the VNS therapy (non-responders).

The statistical analysis of EEG data yielding the responder and non-responder patterns, and optionally also the discriminative electrodes and/or the discriminative intervals typically comprises the following steps:
- for a group of patients (preferably at least 25 patients) with known VNS therapeutic outcome (responder or non-responder), obtaining theta, alpha, beta and gamma frequency bands for at least 10 scalp electrodes from scalp EEG data, said EEG data comprising at least one rest interval, preferably at the beginning of the EEG data, at least one open eyes/close eyes interval, at least one photic stimulation interval, at least one hyperventilation interval, and optionally at least one additional open eyes/close eyes interval and optionally at least one rest interval having the duration of at least 30 s at the end of the EEG data, wherein one interval is selected as the baseline interval,
- obtaining absolute mean powers as mean values of passband power envelope inside the said intervals and inside the baseline interval, and obtaining relative mean powers of the said intervals as the ratio of absolute mean power of the said interval relative to absolute mean power of the baseline interval,
- classifying the data into responder group and non-responder group according to the known VNS therapeutic outcome,
- optionally analyzing the data for the responder and non-responder groups to identify the discriminative electrodes and the discriminative intervals, preferably using the leave-one-out method (alternatively, the discriminative electrodes and/or the discriminative intervals may be determined by the user),
- determining a responder pattern and a non-responder pattern, preferably using classifiers, such as logistic regression (LR) and/or linear support vector machines (SVM) and/or linear discriminant analysis (LDA),
- optionally determining sensitivity, specificity and/or accuracy of the statistical model.

The scalp EEG data used in the statistical analysis are data obtained from the patient before the VNS therapy or VNS implantation. Typically, data from routine scalp EEG recording, which is done for each patient during or after epilepsy diagnosis, may be used in the method of the present invention.

The identification of discriminative electrodes may be performed based on a training data set using feature selection methods, such as stepwise logistic regression. To obtain a robust set of selected electrodes, it is strongly recommended to perform feature selection in leave-one-out (LOO) manner; specifically, if the number of patients in training data set is n, the stepwise logistic regression is performed *n*-times using data of n-1 subjects (i.e. one different subject is left out in each iteration) and then identify the most discriminative electrodes as the ones selected in majority of *n* LOO iterations.

Creation of a classifier based on the selected discriminative electrodes corresponds to estimation of a boundary in multivariate feature space best separating a group of responders from non-responders using classification techniques, such as logistic regression and support vector machines.

Classification of a tested patient into a group of responders or non-responders may be made as follows:
a. classification of the subject based on single classifier, which is trained on all *n* patients from the training data set; or
b. classification of the subject based on majority voting of n classifiers trained on subsets of *n*-1 training patients.

### Brief description of Drawings

Figure 1: Pre-processing of EEG data for analysis
   EEG data record was segmented into eight time intervals, and subsequently filtered into four frequency bands: theta, alpha, beta, and gamma. Further analyses were focused on the oscillatory power changes in these intervals, which were analyzed separately for each frequency band.

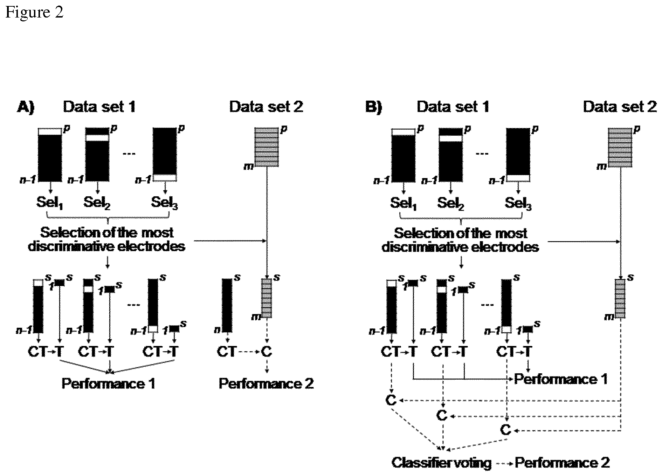

Figure 2: The statistical model for prediction of VNS efficacy based on EEG analysis Schematics representing the two approaches taken for the classification of external data (the data set 2). Equivalent steps taken in each approach are visualized using solid lines. Specifically, selection of the maximally discriminative electrodes from dataset 1 based on *n* LOO iterations is performed. It is then followed by another LOO loop containing n classifier training (CT) and testing (T) sub-experiments leading to a calculation of the performance 1. The two approaches also have in common reduction of the dataset 2 by selection of data corresponding to the maximally discriminative electrode variables identified based on the data set 1. The two approaches differ in the steps represented by dashed lines - i.e. the classification (C) of the reduced dataset 2 using (A) a single classifier trained on all *n* subjects from the dataset 1, and (B) voting of n classifiers trained on n-1 subsets of the dataset 1.
Figure 3: The individual electrodes and groups of electrodes selected for statistical model Individual electrodes (first row) and groups of electrodes (second row) selected for the development of the statistical model, in each time interval and frequency band.

### Examples

### Patient data

The inventors have retrospectively analyzed a group of adult patients implanted with vagal nerve stimulator device for drug-resistant epilepsy in Brno Epilepsy Center between 2005 and 2013. Routine preoperative scalp EEG record according to 10-20 international system, i.e. with standard eyes opening and closing, photic stimulation (PS), and hyperventilation (HV), was tracked down for each patient.

Based on their individual responses to VNS, patients were categorized as responders and non-responders, or as predominant responders or predominant non-responders (i.e. patients who changed their classification category during follow-up).

The study was conducted in St. Anne's University Hospital and approved by local ethic committee. All patients gave their informed consent with the study.

### Patients' description

All patients with drug-resistant epilepsy were implanted with vagal nerve stimulation system, Cyberonics, Houston, according to standard implantation procedure (Landre. Vagus nerve stimulation and refractory partial epilepsies. Rev Neurol 2004;160:S280-287). Before VNS implantation, all patients underwent comprehensive evaluation protocol for epilepsy surgery candidates. Routine scalp EEG, Brain Magnetic Resonance Imaging (MRI), long-term video EEG monitoring, and neuropsychology were done standardly in all patients. Other methods (positron emission tomography [PET], interictal/ictal single photon emission tomography [SPECT], single photon emission tomography co-registered to MRI [SISCOM], and invasive EEG [IEEG]) were done only in some of patients fully based on clinical decision in specific case. Into this study we included only patients who fulfilled following criteria: the duration of VNS treatment was at least 2 years; the efficacy of VNS treatment was determined in regular visits every 3 or 6 months; and the preoperative good quality interictal EEG with eye opening and closing, PS and HV was available for analysis. Patients with co-existence of psychogennic non-epileptic seizures and non-epileptic attacks that mimic epilepsy and could not be reliably distinguished from epileptic seizures were excluded from the study.

The demographic data and data regarding type and number of antiepileptic drugs (AEDs) at the time of vagal nerve stimulator implantation were obtained by review of patients' charts. The efficacy of VNS was categorized using classification system reported by McHugh (McHugh JC, Singh HW, Phillips J, Murphy K, Doherty CP, Delanty N. Outcome measurement after vagal nerve stimulation therapy: proposal of a new classification. Epilepsia 2007;48: 375-8). The cut-off value for seizure-reduction between responders and non-responders was 50%. The patients were defined as responders, respectively non-responders, only if the patient was categorized as a responder or a non-responder for the whole follow-up period. Patients, who changed the classification category during follow-up, were categorized as predominant responders (i.e. patients were responders for more than half of follow-up period) or as predominant non-responders (i.e. patients were non-responders for more than half of follow-up period).

### EEG recording

Interictal scalp EEG was recorded on the 64-channel Alien Deymed system with international 10-20 electrodes placement and sampling frequency of 128 Hz. Standard antialiasing filters (e.g., 50 Hz band filter) were used before digitalization. Occasional artefacts were rejected manually and further processing was performed with artefact-free EEG periods.

The EEG was recorded with the following rests, activities and stimulations: (1) resting state 1 (resting state at the beginning of EEG recording, which is used as a baseline for comparison to other periods); (2) eyes opening/eyes closing; (3) resting state 2 (resting state after eyes closure); (4) photic stimulation; (5) hyperventilation; (6) eyes opening/eyes closing; (7) resting state 3 (resting state after eyes closure); (8) resting state 4.

### EEG analysis

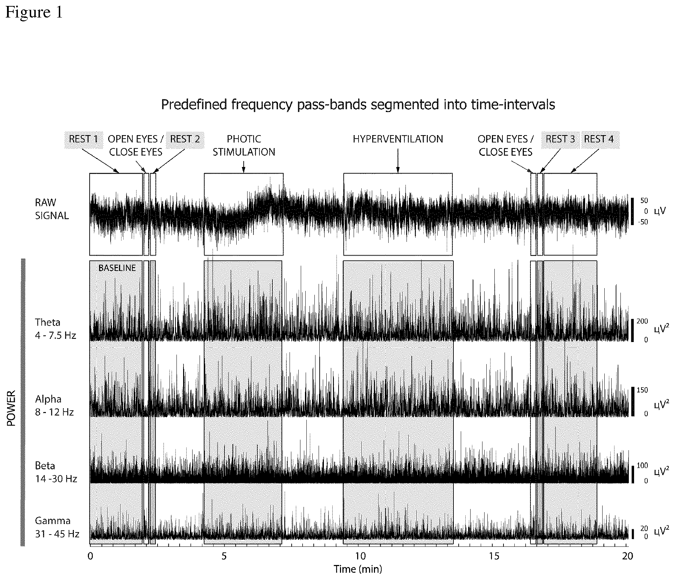

The EEG signals were filtered into four frequency bands: theta (4-7.5 Hz), alpha (8-12 Hz), beta (14-30 Hz), and gamma (31-45 Hz). A Hilbert transform was used to estimate the envelopes of predefined pass-bands frequency oscillations as a function of time (Figure 1). EEG records were segmented into following time-intervals:
1/ Interval 1 - REST 1 - duration 2 minutes
2/ Interval 2 - Eyes opening - eyes closing - duration 10 seconds
3/ Interval 3 - REST 2 (immediately after eyes closure) - duration 10 seconds
4/ Interval 4 - Photic stimulation - duration 2.5 minutes
5/ Interval 5 - Hyperventilation - duration 4 minutes
6/ Interval 6 - Eyes opening - eyes closing - duration 10 seconds
7/ Interval 7 - REST 3 (immediately after eyes closure) - duration 10 seconds
8/ Interval 8 - REST 4 - duration 2 minutes

Further analysis was focused on the oscillatory power changes in these intervals. Absolute mean power of the EEG spectrum was computed as mean value of the passband envelope inside each interval separately for each scalp electrode. Subsequently relative mean power was calculated in terms of event-related desynchronization (ERD)/event-related synchronization (ERS) which is determined as a decrease/increase of power in the frequency band related to the baseline. We selected Interval 1 (REST 1) as a baseline (Pfurtscheller G, Aranibar A. Event-related cortical desynchronization detected by power measurements of scalp EEG. Electroencephalogr Clin Neurophysiol 1977;42: 817-26). Afterwards, we evaluated differences between responders and non-responders by the comparison of relative powers in previously characterized 8 intervals.

### Prediction of response to VNS - statistical model

The statistical model for prediction of VNS efficacy was based on selection of individual electrodes from data of n responders and non-responders using stepwise logistic regression performed in leave-one-out (LOO) manner followed by LOO cross-validated classification using logistic regression (LR), linear support vector machines (SVM) and linear discriminant analysis (LDA). The achieved classification accuracies were compared with the classification by chance using one-sample binomial test.

We also tried to predict VNS efficacy using the same statistical concept, but instead of working with single electrodes we worked with mean values of seven anatomical areas consisting of following grouped electrodes: (1) Fp1, F3, Fz; (2) Fp2, F4, Fz; (3) F7, T3; (4) F8, T4; (5) C3, Cz, C4; (6) P3, Pz, T5, O1; (7) P4, Pz, T6, O2.

Moreover, the developed statistical model with the most successful classifier (LR) was used for classification of *m* predominant responders and non-responders using two approaches (Figure 2). Both approaches began with selection of data corresponding to the most discriminative electrodes. Then, classification of the *m* subjects into a group of responders or non-responders based on single LR classifier, which was trained on all *n* subjects, was performed in the first approach. Whereas in the second approach, the data were classified using majority voting of *n* LR classifiers trained on n-1 data subsets.

### Results

### Participants

We included 60 patients into our study categorized as responders vs. non-responders and 20 patients who were categorized as predominant responders and predominant non-responders. The patients were subdivided into 35 (58%) responders and 25 (42%) non-responders; respectively, into 9 (45%) predominant responders and 11 (55%) predominant non-responders.

### Prediction of VNS response - statistical model

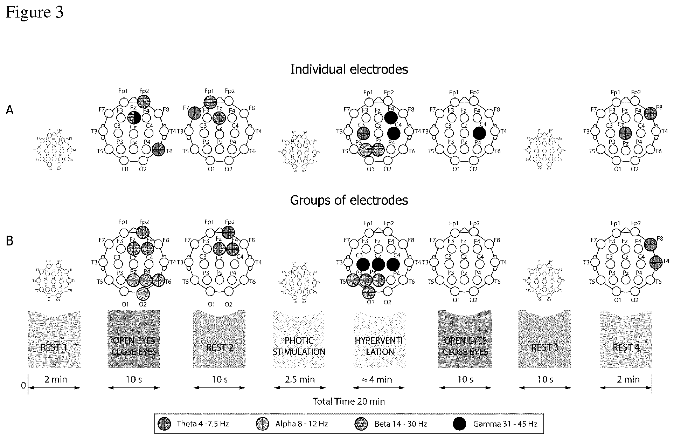

The relative power in 60 patients, who were classified as responders or as non-responders, was used for development of statistical model. The best classification results were obtained using logistic regression classifier based on 13 or 16 selected single electrodes (Figure 3), specifically 90.0% accuracy (88.6% sensitivity, 92.0% specificity) and 90% accuracy (94.3% sensitivity, 84.0% specificity), respectively. The classification accuracies were statistically significantly higher than classification by chance (Table 1). Classification based on groups of electrodes and logistic regression led to slightly lower performance of 86.7% accuracy (88.6% sensitivity, 84.0% specificity). Lowest classification performance measures were obtained in classification using LDA based on selected single as well as grouped features (accuracy around 68.0%).

**Table 1: Classification performance measures for selected 13 and 16 single electrode variables (out of 532 ones) and for 8 grouped electrode variables (out of 196 ones).**

| | 13 single electrode variables | | | | 16 single electrode variables | | | | 8 grouped electrode variables | | | |
|---|---|---|---|---|---|---|---|---|---|---|---|---|
| | acc | sens | spec | p | acc | sens | spec | p | acc | sens | spec | p |
| LR | 90.0 | 88.6 | 92.0 | <0.001 | 90.0 | 94.3 | 84.0 | <0.001 | 86.7 | 88.6 | 84.0 | <0.001 |
| SVM | 83.3 | 85.7 | 80.0 | <0.001 | 85.0 | 85.7 | 84.0 | <0.001 | 75.0 | 62.9 | 92.0 | 0.004 |
| LDA | 70.0 | 54.3 | 92.0 | 0.033 | 68.3 | 54.3 | 88.0 | 0.058 | 65.0 | 48.6 | 88.0 | 0.147 |

| | | | | | | | | | | | | |
|---|---|---|---|---|---|---|---|---|---|---|---|---|
| acc - accuracy, LDA - linear discriminant analysis, LR - logistic regression, p - p-value calculated using one-sample binominal test, sens - sensitivity, spec - specificity, SVM - linear support vector machines | | | | | | | | | | | | |

### Classification of patients according to their predominant response to VNS

As mentioned above, 20 patients with critical changes in their efficacy to VNS during follow-up were excluded from the main classification.

When testing our statistical model for prediction of VNS efficacy in these problematic patients, the accuracy was still equal to 80.0% (with sensitivity 77.8% and specificity 81.8%) while using 13 or as 16 single electrodes and the single LR classifier as well as the classifier voting. In case of grouped electrodes, the classification performance was lower, specifically 75.0% accuracy (77.8% sensitivity, 72.7% specificity) in the case of the single LR classifier and 65.0% accuracy (77.8% sensitivity, 54.5% specificity) using the classifier voting.

## Claims

1. A method for determining of clinical response of a patient suffering from epilepsy to chronic vagal nerve stimulation (VNS) therapy from scalp EEG data, comprising the steps of:
- obtaining theta, alpha, beta and gamma frequency bands for at least one scalp electrode from scalp EEG data obtained from the patient using an EEG recording protocol, said EEG data comprising at least one rest interval, at least one open eyes/close eyes interval, at least one photic stimulation interval, at least one hyperventilation interval, wherein one interval is selected as a baseline interval, and further **characterized in** the steps of:
- obtaining absolute mean powers as mean values of passband power envelope inside at least one discriminative interval and inside the baseline interval, and obtaining the relative mean power of the at least one discriminative interval as the ratio of the absolute mean power of said discriminative interval relative to the absolute mean power of the baseline interval,
- determining from the relative mean powers of the at least one discriminative interval for at least one discriminative electrode whether the patient is a responder or a non-responder to vagal nerve stimulation therapy based on a responder pattern or a non-responder pattern, wherein the responder and non-responder patterns are determined by statistical analysis of EEG data recorded using the said EEG recording protocol for a group of known responders and non-responders.

2. The method according to claim 1, wherein the intervals comprised in the said EEG data further include at least one additional open eyes/close eyes interval and/or at least one rest interval having the duration of at least 30 s at the end of the EEG data.

3. The method according to claim 1 or 2, wherein the baseline interval is a rest interval.

4. The method according to claim 3, wherein the rest interval is a rest interval at the beginning of the EEG data or at the end of the EEG data.

5. The method according to any one of claims 1 to 4, wherein the discriminative electrodes and/or the discriminative intervals are determined by statistical analysis of EEG data recorded using the said EEG recording protocol for a group of known responders and non-responders.

6. The method according to any one of claims 1 to 4, wherein the discriminative electrodes andor the discriminative intervals are selected by a user.

7. The method according to any one of claims 1 to 4, wherein the theta, alpha, beta and gamma frequency bands are obtained for at least 10 scalp electrodes.

8. The method according to any one of claims 1 to 4, wherein the theta, alpha, beta and gamma frequency bands are obtained for at least 13 or at least 16 scalp electrodes.

9. The method according to any one of claims 1 to 8, wherein the discriminative electrode is a single electrode or a group of electrodes, such as electrodes placed in one anatomical area.

10. The method according to claim 9, wherein the groups of electrodes are: (1) Fp1, F3, Fz; (2) Fp2, F4, Fz; (3) F7, T3; (4) F8, T4; (5) C3, Cz, C4; (6) P3, Pz, T5, O1; (7) P4, Pz, T6, O2. Electrodes may also be grouped differently than by anatomical areas.

11. The method according to any one of the preceding claims, wherein the statistical analysis of EEG data yielding the responder and non-responder patterns comprises the following steps:
- for a group of patients with known VNS therapeutic outcome, obtaining theta, alpha, beta and gamma frequency bands for at least one scalp electrode from scalp EEG data obtained from the patients prior to the VNS therapy, said EEG data comprising at least one rest interval, at least one open eyes/close eyes interval, at least one photic stimulation interval, at least one hyperventilation interval, wherein one interval is selected as the baseline interval,
- obtaining absolute mean powers as mean values of passband power envelope inside the said intervals and inside the baseline interval, and obtaining relative mean powers of the said intervals as the ratio of absolute mean power of the said interval relative to absolute mean power of the baseline interval,
- classifying the data into responder group and non-responder group according to the known VNS therapeutic outcome,
- determining a responder pattern and a non-responder pattern, preferably using classifiers, such as logistic regression (LR) and/or linear support vector machines (SVM) and/or linear discriminant analysis (LDA).

12. The method according to claim 11, wherein the statistical analysis of the EEG data also yields the discriminative electrodes and/or the discriminative intervals, and further comprises a step of analyzing the data for the responder and non-responder groups to identify the discriminative electrodes and/or the discriminative intervals using the leave-one-out method after the step of classifying the data into responder group and non-responder group according to the known VNS therapeutic outcome.

13. The method according to claim 11 or 12, wherein the group of patient comprises at least 25 patients.

14. The method according to claim 13, wherein classification of the tested patient into the group of responders or non-responders is made as follows:
- classification of the subject based on single classifier which is trained on all n patients from the training data set; or
- classification of the subject based on majority voting of n classifiers trained on subsets of *n*-1 training patients.

## Patentansprüche

1. Ein Verfahren zur Bestimmung des klinischen Ansprechens eines an Epilepsie leidenden Patienten auf eine Therapie mit chronischer Vagusnervstimulation (VNS) aus Kopfhaut-EEG-Daten, umfassend die Schritte:
- Erhalten von Theta, Alpha, Beta und Gamma-Frequenzbändern für mindestens eine Kopfhautelektrode aus Kopfhaut-EEG-Daten, die vom Patienten unter Verwendung eines EEG-Aufzeichnungsprotokolls erhalten wurden, wobei die EEG-Daten umfassen mindestens ein Ruheintervall, mindestens ein Intervall für offene Augen / geschlossene Augen, mindestens ein photisches Stimulationsintervall und mindestens ein Hyperventilationsintervall, wobei ein Intervall als Referenzintervall ausgewählt ist,
und weiter charakterisiert in den Schritten von:
- Erhalten absoluter mittlerer Leistungen als Mittelwerte der Durchlassbandleistungshüllkurve innerhalb mindestens eines Unterscheidungsintervalls und innerhalb des Referenzintervalls und Erhalten der relativen mittleren Leistung des mindestens einen Unterscheidungsintervalls als Verhältnis der absoluten mittleren Leistung des relativen Unterscheidungsintervalls zur absoluten mittleren Leistung des Referenzintervalls,
- Bestimmen aus den relativen mittleren Leistungen des mindestens einen Unterscheidungsintervalls für mindestens eine Unterscheidungselektrode, ob der Patient ein Responder oder ein Non-Responder auf eine Vagusnervstimulationstherapie ist, basierend auf einem Responder-Muster oder einem Non-Responder-Muster, wobei das Responder- und Non-Responder-Muster werden durch statistische Analyse von EEG-Daten bestimmt, die unter Verwendung des EEG-Aufzeichnungsprotokolls für eine Gruppe bekannter Responder und Non-Responder aufgezeichnet wurden.

2. Verfahren nach Anspruch 1, wobei die in den EEG-Daten enthaltenen Intervalle ferner mindestens ein zusätzliches Intervall für offene Augen / geschlossene Augen und/oder mindestens ein Ruheintervall mit der Dauer von mindestens 30 s am Ende von die EEG-Daten umfassen.

3. Verfahren nach Anspruch 1 oder 2, wobei das Referenzintervall ein Ruheintervall ist.

4. Verfahren nach Anspruch 3, wobei das Ruheintervall ein Ruheintervall am Anfang der EEG-Daten oder am Ende der EEG-Daten ist.

5. Verfahren nach einem der Ansprüche 1 bis 4, wobei die Unterscheidungselektroden und/oder die Unterscheidungsintervalle bestimmt werden durch statistische Analyse von EEG-Daten, die unter Verwendung des EEG-Aufzeichnungsprotokolls für eine Gruppe bekannter Responder und Non-Responder aufgezeichnet wurden.

6. Verfahren nach einem der Ansprüche 1 bis 4, wobei die Unterscheidungselektroden und/oder die Unterscheidungsintervalle von einem Benutzer ausgewählt werden.

7. Verfahren nach einem der Ansprüche 1 bis 4, wobei die Theta-, Alpha-, Beta- und Gamma-Frequenzbänder für mindestens 10 Kopfhautelektroden erhalten werden.

8. Verfahren nach einem der Ansprüche 1 bis 4, wobei die Theta-, Alpha-, Beta- und Gamma-Frequenzbänder für mindestens 13 oder mindestens 16 Kopfhautelektroden erhalten werden.

9. Verfahren nach einem der Ansprüche 1 bis 8, wobei die Unterscheidungselektrode eine einzelne Elektrode oder eine Gruppe von Elektroden ist, wie beispielsweise Elektroden, die in einem anatomischen Bereich angeordnet sind.

10. Verfahren nach Anspruch 9, wobei die Elektrodengruppen sind: (1) Fp1, F3, Fz; (2) Fp2, F4, Fz; (3) F7, T3; (4) F8, T4; (5) C3, Cz, C4; (6) P3, Pz, T5, 01; (7) P4, Pz, T6, 02, oder elektroden können auch anders gruppiert sein als nach anatomischen Bereichen.

11. Verfahren nach einem der vorhergehenden Ansprüche, wobei die statistische Analyse von EEG-Daten, die das Responder- und das Non-Responder-Muster ergeben, die folgenden Schritte umfasst:
- für eine Gruppe von Patienten mit bekanntem therapeutischem VNS-Ergebnis, die Theta-, Alpha-, Beta- und Gamma-Frequenzbänder für mindestens eine Kopfhautelektrode erhalten aus Kopfhaut-EEG-Daten, die von den Patienten vor der VNS-Therapie erhalten wurden, wobei die EEG-Daten mindestens ein Ruheintervall, mindestens ein Intervall für offene Augen / geschlossene Augen, mindestens ein photisches Stimulationsintervall und mindestens ein Hyperventilationsintervall, wobei ein Intervall als Referenzintervall ausgewählt ist,
- Erhalten absoluter mittlerer Leistungen als Mittelwerte der Durchlassbandleistungshüllkurve innerhalb mindestens eines Unterscheidungsintervalls und innerhalb des Referenzintervalls und Erhalten der relativen mittleren Leistung des mindestens einen Unterscheidungsintervalls als Verhältnis der absoluten mittleren Leistung des relativen Unterscheidungsintervalls zur absoluten mittleren Leistung des Referenzintervalls,
- Einteilung der Daten in Respondergruppen und Nicht-Respondergruppen gemäß dem bekannten therapeutischen Ergebnis des VNS;
- Bestimmen eines Responder-Musters und eines Non-Responder-Musters, vorzugsweise unter Verwendung von Klassifizierern wie logistischer Regression (LR) und/oder linearen Unterstützungsvektormaschinen (SVM) und/oder linearer Diskriminanzanalyse (LDA).

12. Verfahren nach Anspruch 11, wobei die statistische Analyse der EEG-Daten auch die Unterscheidungselektroden und/oder die Unterscheidungsintervalle ergibt, und ferner einen Schritt des Analysierens der Daten für die Respondergruppen und Nicht-Respondergruppen umfasst, um die Unterscheidungselektroden und/oder die Unterscheidungsintervalle zu identifizieren unter Verwendung der Auslassmethode nach dem Schritt der Klassifizierung der Daten in Respondergruppen und Nicht-Respondergruppen gemäß dem bekannten therapeutischen VNS-Ergebnis.

13. Verfahren nach Anspruch 11 oder 12, wobei die Patientengruppe mindestens 25 Patienten umfasst.

14. Verfahren nach Anspruch 13, wobei die Klassifizierung des getesteten Patienten in die Gruppe von Respondern oder Non-Respondern wie folgt erfolgt:
- Klassifizierung des Subjekts anhand eines einzelnen Klassifikators, der für alle n Patienten aus dem Trainingsdatensatz trainiert wird; oder
- Klassifizierung des Subjekts basierend auf der Mehrheitsentscheidung von n Klassifikatoren, die in Untergruppen von n-1 Trainingspatienten trainiert wurden.

## Revendications

1. Procédé pour déterminer la réponse clinique d'un patient souffrant d'épilepsie à une thérapie de stimulation vagale chronique (VNS) à partir de données EEG du cuir chevelu, ledit procédé comprenant les étapes de:
- obtenir des bandes de fréquences thêta, alpha, bêta et gamma pour au moins une électrode du cuir chevelu, à partir des données EEG du cuir chevelu obtenues auprès du patient en utilisant un protocole d'enregistrement EEG, lesdites données EEG comprenant au moins un intervalle de repos, au moins un intervalle yeux ouverts / yeux fermés , au moins un intervalle de stimulation photique, au moins un intervalle d'hyperventilation, où un intervalle est sélectionné comme intervalle de référence,
ledit procédé étant **caractérisé en outre par** les étapes de:
- obtenir des puissances moyennes absolues en tant que valeurs moyennes de l'enveloppe de puissance de bande passante à l'intérieur d'au moins un intervalle discriminant et à l'intérieur de l'intervalle de référence, et obtenir la puissance moyenne relative de l'au moins un intervalle discriminant en tant que rapport de la puissance moyenne absolue dudit intervalle discriminant relatif à la puissance moyenne absolue de l'intervalle de référence,
- déterminer à partir des puissances moyennes relatives de l'au moins un intervalle discriminant pour au moins une électrode discriminante si le patient est un répondeur ou un non-répondeur à une thérapie de stimulation vagale sur la base d'un motif répondeur ou d'un motif non-répondeur, où les profils de répondeur et de non-répondeur sont déterminés par analyse statistique des données EEG enregistrées en utilisant ledit protocole d'enregistrement EEG pour un groupe de répondeurs et de non-répondeurs connus.

2. Procédé selon la revendication 1, où les intervalles compris dans lesdites données EEG comprennent en outre au moins un intervalle yeux ouverts / yeux fermés supplémentaire et/ou au moins un intervalle de repos ayant une durée d'au moins 30 s à la fin des données EEG.

3. Procédé selon la revendication 1 ou 2, où l'intervalle de référence est un intervalle de repos.

4. Procédé selon la revendication 3, où l'intervalle de repos est un intervalle de repos au début des données EEG ou à la fin des données EEG.

5. Procédé selon l'une quelconque des revendications 1 à 4, où les électrodes discriminantes et/ou les intervalles discriminants sont déterminés par analyse statistique des données EEG enregistrées en utilisant ledit protocole d'enregistrement EEG pour un groupe de répondeurs et de non-répondeurs connus.

6. Procédé selon l'une quelconque des revendications 1 à 4, où les électrodes discriminantes et/ou les intervalles discriminants sont sélectionnés par un utilisateur.

7. Procédé selon l'une quelconque des revendications 1 à 4, où les bandes de fréquences thêta, alpha, bêta et gamma sont obtenues pour au moins 10 électrodes de cuir chevelu.

8. Procédé selon l'une quelconque des revendications 1 à 4, où les bandes de fréquences thêta, alpha, bêta et gamma sont obtenues pour au moins 13 ou au moins 16 électrodes du cuir chevelu.

9. Procédé selon l'une quelconque des revendications 1 à 8, où l'électrode discriminante est une électrode unique ou un groupe d'électrodes, par exemple des électrodes placées dans une zone anatomique.

10. Procédé selon la revendication 9, où les groupes d'électrodes sont: (1) Fp1, F3, Fz; (2) Fp2, F4, Fz; (3) F7, T3; (4) F8, T4; (5) C3, Cz, C4; (6) P3, Pz, T5, 01; (7) P4, Pz, T6, 02, ou les électrodes peuvent également être regroupées différemment que par zones anatomiques.

11. Procédé selon l'une quelconque des revendications précédentes, où l'analyse statistique des données EEG donnant les motifs répondeur et non-répondeur comprend les étapes suivantes:
- pour un groupe de patients dont le résultat thérapeutique de VNS est connu, obtenir des bandes de fréquences thêta, alpha, bêta et gamma pour au moins une électrode du cuir chevelu à partir des données EEG du cuir chevelu obtenues auprès des patients avant la thérapie VNS, lesdites données EEG comprenant au moins un repos intervalle, au moins un intervalle yeux ouverts / yeux fermés, au moins un intervalle de stimulation photique, au moins un intervalle d'hyperventilation, dans lequel un intervalle est sélectionné comme intervalle de référence,
- obtenir des puissances moyennes absolues en tant que valeurs moyennes de l'enveloppe de puissance de bande passante à l'intérieur desdits intervalles et à l'intérieur de l'intervalle de référence, et obtenir des puissances moyennes relatives desdits intervalles en tant que rapport de la puissance moyenne absolue dudit intervalle par rapport à la puissance moyenne absolue de l'intervalle de référence,
- classer les données en groupe répondeur et groupe non-répondeur selon le résultat thérapeutique de VNS connu,
- déterminer un motif répondeur et un motif non-répondeur, de préférence à l'aide de classificateurs, tels que la régression logistique (LR) et/ou les machines à vecteur de support linéaire (SVM) et/ou l'analyse discriminante linéaire (LDA).

12. Procédé selon la revendication 11, où l'analyse statistique des données EEG donne également les électrodes discriminantes et/ou les intervalles discriminants, et comprend en outre une étape d'analyse des données pour les groupes répondeur et non-répondeur pour identifier le discriminants des électrodes discriminants et/ou des intervalles discriminants en utilisant la méthode du leave-one-out après l'étape de classification des données en groupe répondeur et groupe non.répondeur selon le résultat thérapeutique de VNS connu.

13. Procédé selon la revendication 11 ou 12, où le groupe de patients comprend au moins 25 patients.

14. Procédé selon la revendication 13, où la classification du patient testé dans le groupe des répondeurs ou des non-répondeurs est effectuée comme suit:
- classification du sujet sur la base d'un classificateur unique qui est formé sur les n patients de l'ensemble de données de formation; ou
- classification du sujet sur la base du vote majoritaire de n classificateurs formés sur des sous-ensembles de *n*-1 patients de formation.
